# EUROPEAN PATENT APPLICATION

(11) **EP 4 832 008 A1**
(43) Date of publication of application: **28.10.2026**
(21) Application number: 26157731.6
(22) Date of filing: 11.02.2026
(51) Int. Cl.: G16H 50/20, G16H 50/30

(54) **COMPUTER-IMPLEMENTED METHOD AND APPARATUS FOR PREDICTING PROGRESSION TREND OF RARE DISEASE, ELECTRONIC DEVICE, AND MEDIUM**

(30) Priority: 25.04.2025 CN 202510526018
(71) Applicant: Peking Union Medical College Hospital, Chinese Academy of Medical Sciences & Peking Union Medical College, Beijing 100730 (CN); Digital Health China Technologies Co., Ltd., Beijing 100080 (CN)
(72) Inventor: ZHANG, Shuyang, BEIJING, 100730 (CN); GUO, Jian, BEIJING, 100730 (CN); JIN, Ye, BEIJING, 100730 (CN); GONG, Mengchun, BEIJING, 100080 (CN); SHI, Wenzhao, BEIJING, 100080 (CN); ZHENG, Xihong, BEIJING, 100080 (CN); LIU, Peng, BEIJING, 100730 (CN)
(74) Representative: Chimini, Francesco

(57) **Abstract**

The present invention relates to the field of Internet technologies, and specifically to a computer-implemented method and an apparatus for predicting a progression trend of a rare disease, an electronic device, and a medium. The computer-implemented method includes: partitioning all medical examination data based on data characteristics of all the medical examination data in a current time period, so as to obtain medical examination data in multiple target time periods; inputting the medical examination data in each target time period into a disease index determination model corresponding to the rare disease type, respectively, so as to obtain a disease index corresponding to each target time period; generating a target trend polyline by taking the target time period as abscissa, and corresponding disease index as ordinate; calculating a similarity between the target trend polyline and each reference trend polyline; and determining the progression trend for the rare disease type in the rare disease patient based on the similarity. The present invention enables accurate prediction of the progression trend of the rare disease.

## Description

### TECHNICAL FIELD

The present invention relates to the field of Internet technologies, and specifically to a computer-implemented method and an apparatus for predicting a progression trend of a rare disease, an electronic device, and a medium.

### BACKGROUND ART

Currently, clinicians see a relatively small number of patients with rare diseases, and accumulate limited experience. Inexperienced clinicians find it difficult to accurately predict progression of patients' condition, and fail to take treatment measures for patients in advance, leading to missed optimal treatment opportunity and prolonged treatment duration and cycle for many patients.

Therefore, how to predict a progression trend of a rare disease has become a problem to be urgently solved in the art.

### SUMMARY

In view of this, the present invention aims at providing a computer-implemented method and an apparatus for predicting a progression trend of a rare disease, an electronic device, and a medium, which enable accurate prediction of the progression trend of the rare disease.

In the first aspect, embodiments of the present invention provide a computer-implemented method for predicting a progression trend of a rare disease, including steps of:
acquiring by means of an acquisition module 301 a rare disease type of a target rare disease patient and medical examination data in a current time period;
judging, by means of a judgment module 302, for each time point in the current time period, whether there are any missing medical examination items correspondingly at the time point based on medical examination data at the time point and a set of medical examination items corresponding to the rare disease type, where the missing medical examination items refer to medical examination items without medical examination data;
determining by means of a determination module 303 an imputation weight corresponding to each missing medical examination item at the time point, if there are missing medical examination items correspondingly at the time point, based on a medical examination item association relationship corresponding to the rare disease type and the medical examination data at the time point, where the medical examination item association relationship includes other medical examination items associated with each medical examination item;
determining by means of said determination module 303 a missing medical examination item with a largest imputation weight as a target missing medical examination item at the time point;
inputting, by means of an input module 304, within the medical examination data at the time point, medical examination data corresponding to the other medical examination items associated with the target missing medical examination item at the time point into an imputation model corresponding to the rare disease type and the target missing medical examination item, so as to obtain medical examination data for the target missing medical examination item at the time point; and returning to the step of judging by means of said judgment module 302 whether there are any missing medical examination items correspondingly at the time point based on the medical examination data at the time point and the set of medical examination items corresponding to the rare disease type, so as to continue execution;
partitioning by means of a partitioning module 305 all the medical examination data based on data characteristics of all the medical examination data in the current time period, so as to obtain medical examination data in multiple target time periods;
inputting by means of said input module 304 the medical examination data in each target time period into a disease index determination model corresponding to the rare disease type, respectively, so as to obtain a disease index corresponding to each target time period, where the disease index determination model is trained with medical examination data samples and corresponding disease index labels;
generating by means of a generation module 306 a target trend polyline by taking the target time period as abscissa, and corresponding disease index as ordinate;
calculating by means of a calculation module 307 a similarity between the target trend polyline and each reference trend polyline, where the reference trend polyline is generated based on disease indexes for a patient with the rare disease type in multiple time periods; and
determining by means of said determination module 303 the progression trend for the rare disease type in the rare disease patient based on the similarity.

In a possible embodiment, the medical examination item association relationship corresponding to the rare disease type is determined through steps of:
acquiring by means of said acquisition module 301 historical medical examination data corresponding to each medical examination item in the set of medical examination items from a plurality of patients with the rare disease type at each historical time point;
calculating by means of said calculation module 307 an initial correlation coefficient between each two medical examination items of each patient based on all the historical medical examination data corresponding to each patient;
for each two medical examination items, calculating by means of said calculation module 307 a mean of initial correlation coefficients of all the patients for the two medical examination items, so as to obtain a target correlation coefficient between the two medical examination items; and
determining, by means of said determination module 303 if the target correlation coefficient between the two medical examination items is greater than or equal to a preset correlation coefficient, that an association relationship exists between the two medical examination items.

In a possible embodiment, the step of calculating by means of a calculation module 307 the initial correlation coefficient between each two medical examination items of each patient based on all the historical medical examination data corresponding to each patient includes:
substituting historical medical examination data of each two medical examination items of the patient into a following formula, to calculate the initial correlation coefficient between each two medical examination items of the patient:
   $dCor \left(X, Y\right) = \frac{{dCor}^{2} \left(X, Y\right)}{\sqrt{dVar \left(X\right) ⋅ dVar \left(Y\right)}} ,$
where dCor(X,Y) is an initial correlation coefficient between historical medical examination data of a first medical examination item X in the two medical examination items and historical medical examination data of a second medical examination item Y in the two medical examination items of the patient, dCor²(X,Y) is a distance covariance between the historical medical examination data of the first medical examination item X and the historical medical examination data of the second medical examination item Y of the patient, dVar(X) is a distance variance of the historical medical examination data of the first medical examination item X of the patient, and dVar(Y) is a distance variance of the historical medical examination data of the second medical examination item Y of the patient.

In a possible embodiment, the step of partitioning by means of said partitioning module 305 all the medical examination data based on the data characteristics of all the medical examination data in the current time period, so as to obtain the medical examination data in the multiple target time periods includes:
partitioning by means of said partitioning module 305 all the medical examination data according to a preset partitioning interval, so as to obtain medical examination data in multiple initial time periods;
partitioning, by means of said partitioning module 305 for each initial time period, the medical examination data within the initial time period based on medical examination data of a medical examination item with a largest variance within the initial time period, if a variance of the medical examination data corresponding to any medical examination item within the initial time period is greater than a preset variance, so as to obtain medical examination data within the target time period; and
determining by means of said determination module 303 the medical examination data in the initial time period as the medical examination data in the target time period, if a variance of the medical examination data corresponding to all the medical examination items in the initial time period is less than or equal to the preset variance.

In a possible embodiment, the step of partitioning by means of said partitioning module 305 the medical examination data within the initial time period based on the medical examination data of the medical examination item with the largest variance within the initial time period, so as to obtain the medical examination data within the target time period includes:
determining by means of said determination module 303 a time point of a maximum value as a split time point if the time point of the maximum value in the medical examination data of the medical examination item with the largest variance is neither a latest time point in the initial time period nor an earliest time point in the initial time period;
determining by means of said determination module 303 a time point of a minimum value as a split time point if the time point of the minimum value in the medical examination data of the medical examination item with the largest variance is neither a latest time point in the initial time period nor an earliest time point in the initial time period;
partitioning by means of said partitioning module 305 the medical examination data in the initial time period at the split time point, so as to obtain the medical examination data in the target time period; and
splitting the medical examination data in the initial time period equally if the time point of the maximum value in the medical examination data corresponding to the medical examination item with the largest variance is the latest time point in the initial time period or the earliest time point in the initial time period, and the time point of the minimum value in the medical examination data corresponding to the medical examination item with the largest variance is the latest time point in the initial time period or the earliest time point in the initial time period, so as to obtain medical examination data in two target time periods.

In a possible embodiment, the step of determining by means of said determination module 303 the progression trend for the rare disease type in the rare disease patient based on the similarity includes:
determining by means of said determination module 303 an actual progression trend corresponding to a reference trend polyline with a maximum similarity, if a maximum value of the similarity is greater than or equal to a preset similarity, as the progression trend corresponding to the target rare disease patient;
calculating, by means of a calculation module 307 if the maximum value of the similarity is less than the preset similarity and target points with a tangent slope of 0 exist in the target trend polyline, a difference between a disease index corresponding to a temporally latest target point and a disease index at a temporally latest point in the target trend polyline;
determining by means of said determination module 303 the progression trend corresponding to the rare disease patient as an improving trend if the difference is greater than 0; and
determining by means of said determination module 303 the progression trend corresponding to the rare disease patient as a deteriorating trend if the difference is less than 0.

In a possible embodiment, if the maximum value of the similarity is less than the preset similarity, the computer-implemented method further includes:
determining by means of said determination module 303 the target trend polyline as a reference trend polyline.

In a possible embodiment, the step of determining by means of said determination module 303 an imputation weight corresponding to each missing medical examination item at the time point based on a medical examination item association relationship corresponding to the rare disease type and the medical examination data at the time point comprises:
for each missing medical examination item, the number of other medical examination items associated with the missing medical examination item and having corresponding medical examination data is counted based on the medical examination data at the time point, to obtain the imputation weight corresponding to the missing medical examination item.

In a possible embodiment, the imputation model is a deep learning model trained in advance with medical examination data samples of other medical examination items associated with the missing medical examination item and corresponding medical examination data labels from the same patient at the same time point.

In a possible embodiment, the disease index determination model is a deep learning model trained in advance with medical examination data samples of patients in a target time period corresponding to the rare diseased type and corresponding disease index labels.

In a possible embodiment, the step of calculating by means of a calculation module 307 a similarity between the target trend polyline and each reference trend polyline comprises:
if the target trend polyline is longer than the reference trend polyline, trimming a leading section of the target trend polyline, so as to allow the remaining target trend polyline and the reference trend polyline to be of equal length;
if the target trend polyline is shorter than the reference trend polyline, trimming a leading section of the reference trend polyline, so as to allow the remaining target trend polyline and the reference trend polyline to be of equal length; and
calculating by means of a calculation module 307 the similarity between the target trend polyline and each reference trend polyline based on the trimmed target trend polyline and reference trend polyline through a formula as follows:
   $d = \frac{1}{\sqrt{{\sum }_{i = 1}^{n} {\left({h}_{i}-{g}_{i}\right)}^{2}}} ,$
wherein d is similarity between the target trend polyline and the reference trend polyline, n is length of the target trend polyline, hi is disease index of a point with abscissa i in the trimmed target trend polyline, and gᵢ is disease index of a point with abscissa i in the reference trend polyline.

In a possible embodiment, if the difference is greater than a first preset difference, the progression trend corresponding to the rare disease patient is a rapidly improving trend; and
if the difference is greater than 0 and less than the first preset difference, the progression trend corresponding to the rare disease patient is a slowly improving trend; and/or
if the difference is less than a second preset difference, the progression trend corresponding to the rare disease patient is the rapidly deteriorating trend; and
if the difference is less than 0 and greater than the second preset difference, the progression trend corresponding to the rare disease patient is the slowly deteriorating trend.

In the second aspect, embodiments of the present invention further provide an apparatus for predicting a progression trend of a rare disease, including:
an acquisition module 301, configured to acquire a rare disease type of a target rare disease patient and medical examination data in a current time period;
a judgment module 302, configured to judge, for each time point in the current time period, whether there are any missing medical examination items correspondingly at the time point based on medical examination data at the time point and a set of medical examination items corresponding to the rare disease type, where the missing medical examination items refer to those without medical examination data;
a determination module 303, configured to determine an imputation weight corresponding to each missing medical examination item at the time point, if there are missing medical examination items correspondingly at the time point, based on a medical examination item association relationship corresponding to the rare disease type and the medical examination data at the time point, where the medical examination item association relationship includes other medical examination items associated with each medical examination item;
the determination module 303 being further configured to determine a missing medical examination item with a largest imputation weight as a target missing medical examination item at the time point;
an input module 304, configured to input, within the medical examination data at the time point, medical examination data corresponding to other medical examination items associated with the target missing medical examination item at the time point into an imputation model corresponding to the rare disease type and the target missing medical examination item, so as to obtain medical examination data for the target missing medical examination item at the time point; and return to judge whether there are any missing medical examination items correspondingly at the time point based on the medical examination data at the time point and the set of medical examination items corresponding to the rare disease type, so as to continue execution;
a partitioning module 305, configured to partition all the medical examination data based on data characteristics of all the medical examination data in the current time period, so as to obtain medical examination data in multiple target time periods;
the input module 304 being further configured to input the medical examination data in each target time period into a disease index determination model corresponding to the rare disease type, respectively, so as to obtain the disease index corresponding to each target time period, where the disease index determination model is trained with medical examination data samples and corresponding disease index labels;
a generation module 306, configured to generate a target trend polyline by taking the target time period as abscissa, and corresponding disease index as ordinate;
a calculation module 307, configured to calculate a similarity between the target trend polyline and each reference trend polyline, where the reference trend polyline is generated based on disease indexes for a patient with the rare disease type in multiple time periods; and
the determination module 303 being further configured to determine the progression trend for the rare disease type in the rare disease patient based on the similarity.

In the third aspect, embodiments of the present invention further provide an electronic device, including: a processor, a storage medium, and a bus, where the storage medium stores machine-readable instructions executable by the processor. When the electronic device is running, the processor and the storage medium communicate via the bus, and the processor executes the machine-readable instructions, so as to execute the steps of the computer-implemented method for predicting the progression trend of the rare disease according to any item in the first aspect.

In the fourth aspect, embodiments of the present invention further provide a computer-readable storage medium, where a computer program is stored on the computer-readable storage medium, and the computer program, when run by a processor, executes the steps of the computer-implemented method for predicting the progression trend of the rare disease according to any item in the first aspect.

The embodiments of the present invention provide the computer-implemented method and apparatus for predicting the progression trend of the rare disease, the electronic device, and the medium. The computer-implemented method includes: partitioning by means of said partitioning module 305 all the medical examination data based on data characteristics of all the medical examination data in the current time period, so as to obtain medical examination data in multiple target time periods; inputting by means of said input module 304 the medical examination data in each target time period into the disease index determination model corresponding to the rare disease type, respectively, so as to obtain the disease index corresponding to each target time period; generating by means of said generation module 306 the target trend polyline by taking the target time period as abscissa, and corresponding disease index as ordinate; calculating by means of a calculation module 307 the similarity between the target trend polyline and each reference trend polyline; and determining the progression trend for the rare disease type in the rare disease patient based on the similarity. The present invention enables accurate prediction of the progression trend of the rare disease.

### BRIEF DESCRIPTION OF DRAWINGS

In order to more clearly illustrate technical solutions of embodiments of the present invention, drawings which need to be used in the embodiments will be briefly introduced below. It should be understood that the drawings merely show some embodiments of the present invention, and thus should not be considered as limitation to the scope. Those ordinarily skilled in the art still could obtain other relevant drawings according to these drawings, without making inventive efforts.
FIG. 1 shows a flowchart of a computer-implemented method for predicting a progression trend of a rare disease provided in embodiments of the present invention;
FIG. 2 shows a flowchart for classifying medical examination data provided in embodiments of the present invention;
FIG. 3 shows a structural schematic diagram of an apparatus for predicting a progression trend of a rare disease provided in embodiments of the present invention; and
FIG. 4 is a structural schematic diagram of an electronic device provided in embodiments of the present invention.

### DETAILED DESCRIPTION OF EMBODIMENTS

In order to make objectives, technical solutions, and advantages of embodiments of the present invention clearer, the technical solutions in the embodiments of the present invention will be described clearly and completely below in conjunction with drawings in the embodiments of the present invention. It should be understood that the drawings in the present invention are merely for an illustrative and descriptive purpose, rather than limiting the scope of protection of the present invention. Besides, it should be understood that schematic drawings are not drawn to scale. Flowcharts used in the present invention show operations implemented according to some embodiments of the present invention. It should be understood that the operations in the flowcharts may be implemented out of order, and steps without a logical relationship may be implemented reversely or concurrently. In addition, those skilled in the art could add, guided by contents of the present invention, one or more other operations to the flowcharts, or remove one or more operations from the flowcharts.

Besides, only some but not all embodiments of the present invention are described. Generally, components in the embodiments of the present invention, as described and shown in the drawings herein, can be arranged and designed in various different configurations. Therefore, the following detailed description of the embodiments of the present invention provided in the drawings is not intended to limit the scope claimed in the present invention, but merely illustrates chosen embodiments of the present invention. All of other embodiments obtained by those skilled in the art based on the embodiments of the present invention without making inventive efforts shall fall within the scope of protection of the present invention.

In order to enable those skilled in the art to use the contents of the present invention, in conjunction with a specific application scenario "field of Internet technologies," the following embodiments are provided. For those skilled in the art, general principles defined herein could be applied to other embodiments and application scenarios without departing from the spirit and scope of the present invention. While the present invention is primarily described in the context of the "field of Internet technologies," it should be understood that this is only an exemplary example.

It should be noted that the term "include" to be used in the embodiments of the present invention is intended to indicate presence of feature(s) stated later, but it does not exclude addition of other features.

A computer-implemented method for predicting a progression trend of a rare disease provided in embodiments of the present invention is described in detail below.

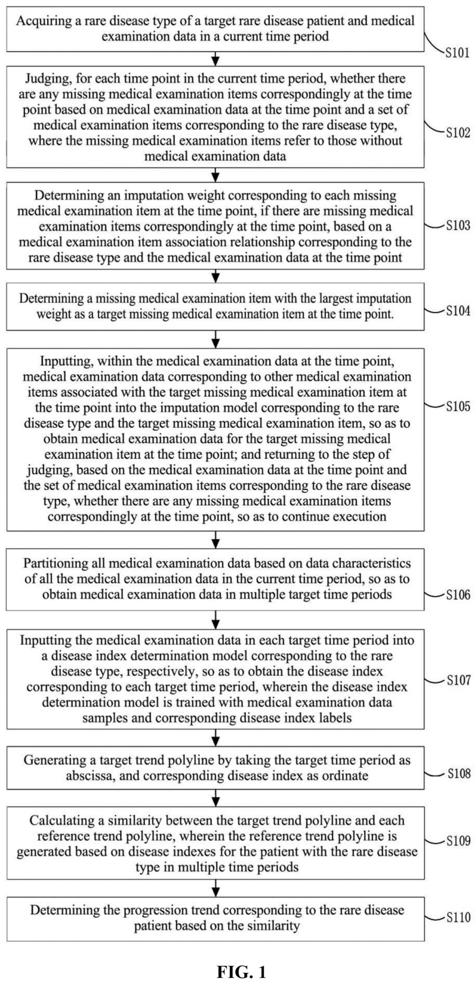

With reference to FIG. 1, it is a flowchart of a computer-implemented method for predicting a progression trend of a rare disease for a target rare disease patient provided in embodiments of the present invention. Various exemplary steps in embodiments of the present invention are illustrated below.

S101: acquiring by means of said acquisition module 301 a rare disease type of a target rare disease patient and medical examination data in a current time period.

In embodiments of the present invention, the target rare disease patient refers to a patient whose progression trend of rare disease needs to be predicted. The rare disease type of the target rare disease patient refers to a rare disease type (such as hemophilia, myasthenia gravis, and neuromyelitis optica) suffered by the target rare disease patient. Duration of the current time period can be one week, half a month, etc., depending on actual situations. The medical examination data can be uploaded to a medical examination database of a medical decision support system by the target rare disease patient during follow-up visit by a physician, or can be uploaded to the medical examination database of the medical decision support system by the physician during follow-up visit to the target rare disease patient. Different rare disease types are corresponding to different sets of medical examination items. The medical examination data in the current time period should be medical examination values of medical examination items (such as blood pressure and blood sugar) in a set of medical examination items corresponding to this rare disease type.

Herein, the medical decision support system is a software development system which can be configured to execute the computer-implemented method for predicting the progression trend of the rare disease provided in the embodiments of the present invention. The medical decision support system stores multi-source data sets (the data can include medical information, papers, rare disease treatment data of patients, etc.), and is featured by a variety of data types (text data, table data, images, videos, etc.), diverse data sources (medical equipment, user input, etc.), a plurality of data owners (such as patients and rare disease specialists), multiple time points, multiple regions and the like, and thus the system is a medical decision support system based on multi-source data.

S102: judging, by means of said judgment module 302for each time point in the current time period, whether there are any missing medical examination items correspondingly at the time point based on medical examination data at the time point and a set of medical examination items corresponding to the rare disease type, where the missing medical examination items refer to those without medical examination data.

In embodiments of the present invention, a medical examination item lacking corresponding medical examination data in the set of medical examination items corresponding to the rare disease type is a missing medical examination item. For example, a set of medical examination items corresponding to a rare disease type includes medical examination item 1, medical examination item 2, and medical examination item 3, and medical examination data corresponding to the time point includes medical examination data for the medical examination item 1 and medical examination data for the medical examination item 2; therefore, the missing medical examination item for the medical examination data at this time point is the medical examination item 3.

S103: determining by means of said determination module 303 an imputation weight corresponding to each missing medical examination item at the time point, if there are missing medical examination items correspondingly at the time point, based on a medical examination item association relationship corresponding to the rare disease type and the medical examination data at the time point.

In embodiments of the present invention, different rare disease types are corresponding to different medical examination item association relationships. The medical examination item association relationship includes other medical examination items associated with each medical examination item, and the number of other medical examination items associated with each medical examination item may be one or more. Specifically, for each missing medical examination item, the number of other medical examination items associated with the missing medical examination item and having corresponding medical examination data is counted based on the medical examination data at the time point, to obtain the imputation weight corresponding to the missing medical examination item. Herein, the medical examination data of other medical examination items can be acquired from the medical examination database in step S101, or can also be imputed by an imputation model. The greater the value corresponding to the imputation weight is, the more accurate the medical examination data obtained by the imputation model is.

In addition, the medical examination item association relationship corresponding to the rare disease type is determined through following steps.

Step 1: acquiring by means of said acquisition module 301 historical medical examination data corresponding to each medical examination item in the set of medical examination items from a plurality of patients with the rare disease type at each historical time point.

For example, the set of medical examination items corresponding to the rare disease type includes medical examination item 1, medical examination item 2, and medical examination item 3. Medical examination data of a plurality of patients with the rare disease type at a plurality of historical time points are acquired from the medical examination database. The medical examination data for each patient at each historical time point includes a corresponding medical examination value of the patient for the medical examination item 1 at the historical time point, a corresponding medical examination value of the patient for the medical examination item 2 at the historical time point, and a corresponding medical examination value of the patient for the medical examination item 3 at the historical time point.

Step 2: calculating by means of a calculation module 307 an initial correlation coefficient between each two medical examination items of each patient based on all historical medical examination data corresponding to each patient.

In embodiments of the present invention, for each patient, an initial correlation coefficient between each two medical examination items of the patient needs to be calculated based on historical medical examination data of the patient. The greater the initial correlation coefficient is, the stronger the association between the two medical examination items is.

For example, there are patient A and patient B; and the set of medical examination items includes medical examination item 1, medical examination item 2, and medical examination item 3. For the patient A, an initial correlation coefficient between the medical examination item 1 and the medical examination item 2 for the patient A is calculated based on historical medical examination data of the patient A; an initial correlation coefficient between the medical examination item 2 and the medical examination item 3 for the patient A is calculated based on the historical medical examination data of the patient A; and an initial correlation coefficient between the medical examination item 1 and the medical examination item 3 for the patient A is calculated based on the historical medical examination data of the patient A. For the patient B, an initial correlation coefficient between the medical examination item 1 and the medical examination item 2 for the patient B is calculated based on historical medical examination data of the patient B; an initial correlation coefficient between the medical examination item 2 and the medical examination item 3 for the patient B is calculated based on the historical medical examination data of the patient B; and an initial correlation coefficient between the medical examination item 1 and the medical examination item 3 for the patient B is calculated based on the historical medical examination data of the patient B.

Specifically, historical medical examination data of each two medical examination items of the patient are substituted into following formulas, to calculate the initial correlation coefficient between each two medical examination items of the patient:
$dCor \left(X, Y\right) = \frac{{dCor}^{2} \left(X, Y\right)}{\sqrt{dVar \left(X\right) ⋅ dVar \left(Y\right)}} ;$
${dCor}^{2} \left(X, Y\right) = \frac{1}{{n}^{2}} {\sum }_{i = 1}^{n} {\sum }_{j = 1}^{n} {{A}_{ij}}^{˜} {{B}_{ij}}^{˜} ;$
$dVar \left(X\right) = \frac{1}{{n}^{2}} {\sum }_{i = 1}^{n} {\sum }_{j = 1}^{n} {{{A}_{ij}}^{˜}}^{2} ;$
$dVar \left(Y\right) = \frac{1}{{n}^{2}} {\sum }_{i = 1}^{n} {\sum }_{j = 1}^{n} {{{B}_{ij}}^{˜}}^{2} ;$
${{A}_{ij}}^{˜} = {A}_{ij} - {a}_{i} - {a}_{j} + \overline{A} ; \overline{A} = \frac{1}{{n}^{2}} {\sum }_{i = 1}^{n} {\sum }_{j = 1}^{n} {A}_{ij} ;$
${{B}_{ij}}^{˜} = {B}_{ij} - {b}_{i} - {b}_{j} + \overline{B} ; \overline{B} = \frac{1}{{n}^{2}} {\sum }_{i = 1}^{n} {\sum }_{j = 1}^{n} {B}_{ij} ;$
${A}_{ij} = \left|{x}_{i}-{x}_{j}\right| ;$ and
${B}_{ij} = \left|{y}_{i}-{y}_{j}\right| ,$
wherein dCor(X,Y) is an initial correlation coefficient between historical medical examination data of a first medical examination item X in two medical examination items and historical medical examination data of a second medical examination item Y in the two medical examination items of the patient, dCor²(X,Y) is a distance covariance between the historical medical examination data of the first medical examination item X and the historical medical examination data of the second medical examination item Y of the patient, dVar(X) is a distance variance of the historical medical examination data of the first medical examination item X of the patient, and dVar(Y) is a distance variance of the historical medical examination data of the second medical examination item Y of the patient; ${{A}_{ij}}^{˜}$ is a numerical value at an i-th row and a j-th column of a doubly centered distance matrix of the historical medical examination data of the first medical examination item X of the patient; ${{B}_{ij}}^{˜}$ is a numerical value at an i-th row and a j-th column in a doubly centered distance matrix of the historical medical examination data of the second medical examination item Y of the patient; Aᵢⱼ is a numerical value at an i-th row and a j-th column in a distance matrix of the historical medical examination data of the first medical examination item X of the patient, and Bᵢⱼ is a numerical value at an i-th row and a j-th column in a distance matrix of the historical medical examination data of the second medical examination item Y of the patient; aᵢ is a mean of numerical values at the i-th row in the distance matrix of the historical medical examination data of the first medical examination item X of the patient, bᵢ is a mean of numerical values at the i-th row in the distance matrix of the historical medical examination data of the second medical examination item Y of the patient, aⱼ is a mean of numerical values at the j-th column in the distance matrix of the historical medical examination data of the first medical examination item X of the patient, bⱼ is a mean of numerical values at the j-th column in the distance matrix of the historical medical examination data of the second medical examination item Y of the patient, xᵢ is an i-th piece of historical medical examination data of the first medical examination item X of the patient, Xⱼ is a j-th piece of historical medical examination data of the first medical examination item X of the patient, yᵢ is an i-th piece of historical medical examination data of the second medical examination item Y of the patient, yⱼ is a j-th piece of historical medical examination data of the second medical examination item Y of the patient, n is the number of elements in the distance matrix of the historical medical examination data of the first medical examination item X or the number of elements in the distance matrix of the historical medical examination data of the second medical examination item Y.

Step 3: for each two medical examination items, calculating by means of a calculation module 307 a mean of initial correlation coefficients of all the patients for the two medical examination items, so as to obtain a target correlation coefficient between the two medical examination items.

For example, for patient A and patient B, regarding the medical examination item 1 and the medical examination item 2, the initial correlation coefficient between the medical examination item 1 and the medical examination item 2 of the patient A and the initial correlation coefficient between the medical examination item 1 and the medical examination item 2 of the patient B are added to obtain a sum, and the sum is divided by 2, so as to obtain the target correlation coefficient between the medical examination item 1 and the medical examination item 2.

Step 4: determining, by means of said determination module 303 if the target correlation coefficient between the two medical examination items is greater than or equal to a preset correlation coefficient, that an association relationship exists between the two medical examination items.

Step 5: if the target correlation coefficient between the two medical examination items is less than the preset correlation coefficient, no association relationship exists between the two medical examination items.

S104: determining by means of said determination module 303 a missing medical examination item with the largest imputation weight as a target missing medical examination item at the time point.

S105: inputting, by means of said input module 304 within the medical examination data at the time point, medical examination data corresponding to other medical examination items associated with the target missing medical examination item at the time point into the imputation model corresponding to the rare disease type and the target missing medical examination item, so as to obtain medical examination data for the target missing medical examination item at the time point; and returning to the step of judging, based on the medical examination data at the time point and the set of medical examination items corresponding to the rare disease type, whether there are any missing medical examination items correspondingly at the time point, so as to continue execution.

In embodiments of the present invention, different rare disease types and target missing medical examination items are corresponding to different imputation models. The imputation models are configured to impute medical examination data corresponding to target missing medical examination items. The imputation model is a deep learning model trained in advance with medical examination data samples of other medical examination items associated with the missing medical examination item and corresponding medical examination data labels from the same patient at the same time point, taking the medical examination data of other medical examination items associated with the missing medical examination item as input, and the medical examination data corresponding to the missing medical examination item as output.

Here, to avoid inaccuracy in subsequent progression trend prediction caused by potentially incomplete medical examination data stored in the medical examination database, the present invention further imputes the medical examination data. In addition, in the present invention, upon imputing the medical examination data corresponding to one missing medical examination item, the imputation weights of other missing medical examination items are re-determined, so as to further impute the missing medical examination item with the largest imputation weight, thereby improving the accuracy of medical examination data imputed.

S106: partitioning by means of said partitioning module 305 all medical examination data based on data characteristics of all the medical examination data in the current time period, so as to obtain medical examination data in multiple target time periods.

In embodiments of the present invention, all the medical examination data in the current time period include those acquired from the medical examination database in step S101 and those imputed by the imputation model. A numerical dispersion degree of the medical examination data within the target time periods should satisfy preset dispersion degree conditions, and specific preset dispersion degree conditions can be defined based on practical circumstances, such as a mean, a variance or a mean deviation, so that a disease index subsequently determined corresponding to a target time period can represent an overall condition in that target time period.

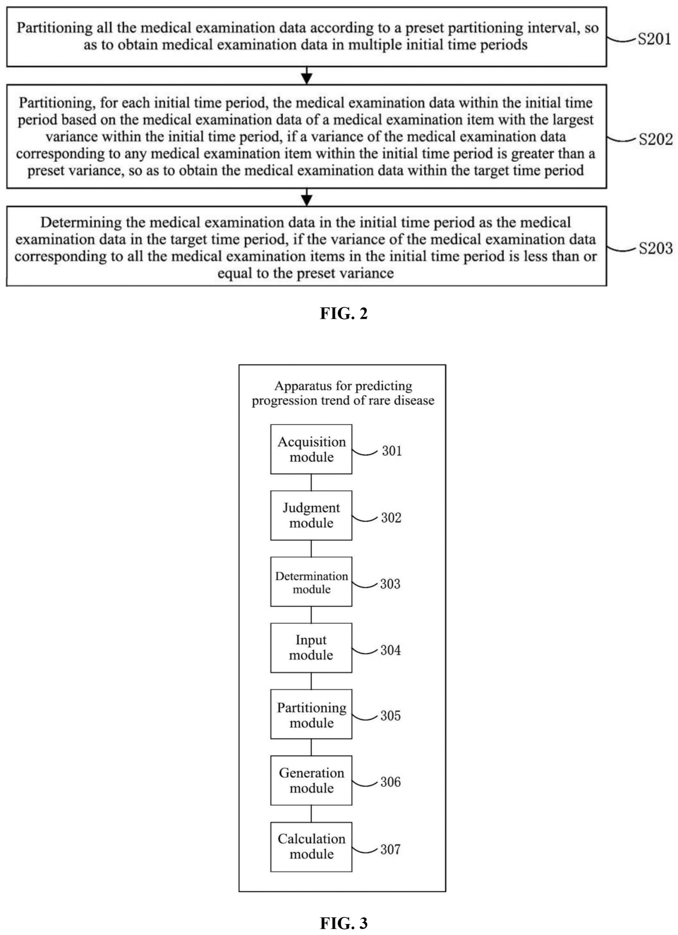

Specifically, with reference to FIG. 2, it is a flowchart for partitioning medical examination data provided in embodiments of the present invention. Various exemplary steps in embodiments of the present invention are illustrated below.

S201: partitioning by means of said partitioning module 305 all the medical examination data according to a preset partitioning interval, so as to obtain medical examination data in multiple initial time periods.

In embodiments of the present invention, preset interval can be set according to practical circumstances, such as one day and two days. Through partitioning according to the preset interval, the numerical dispersion degree of the medical examination data in each time period can be effectively reduced.

For example, the medical examination data within the current time period is medical examination data within one week, and the preset interval is one day, then partitioning by means of said partitioning module 305 the medical examination data yields medical examination data from day 0 to day 1, medical examination data from day 1 to day 2, medical examination data from day 2 to day 3, medical examination data from day 3 to day 4, medical examination data from day 4 to day 5, medical examination data from day 5 to day 6, and medical examination data from day 6 to day 7.

S202: partitioning, by means of said partitioning module 305 for each initial time period, the medical examination data within the initial time period based on the medical examination data of a medical examination item with the largest variance within the initial time period, if a variance of the medical examination data corresponding to any medical examination item within the initial time period is greater than a preset variance, so as to obtain the medical examination data within the target time period.

Specifically, the medical examination data within the initial time period are partitioned based on the medical examination data corresponding to the medical examination item with the largest variance within that initial time period through following steps.
a: determining by means of said determination module 303 a time point of a maximum value as a split time point if the time point of the maximum value in the corresponding medical examination data of the medical examination item with the largest variance is neither a latest time point in the initial time period nor an earliest time point in the initial time period.

For example, the medical examination item with the largest variance in the initial time period is medical examination item 2, the initial time period contains medical examination data at 3:00, medical examination data at 4:00, medical examination data at 7:00, medical examination data at 8:00, medical examination data at 9:00, and medical examination data at 12:00, the maximum value in the medical examination data of the medical examination item 2 is the medical examination data at 4:00, while 4:00 is neither the latest time 12:00 with corresponding medical examination data in the initial time period, nor the earliest time 3:00 with corresponding medical examination data in the initial time period, and then the time point of the maximum value is determined as the split time point.

b: determining by means of said determination module 303 a time point of a minimum value as a split time point if the time point of the minimum value in the corresponding medical examination data of the medical examination item with the largest variance is neither a latest time point in the initial time period nor an earliest time point in the initial time period.

For example, the medical examination item with the largest variance in the initial time period is medical examination item 2, the initial time period contains medical examination data at 3:00, medical examination data at 4:00, medical examination data at 7:00, medical examination data at 8:00, medical examination data at 9:00, and medical examination data at 12:00, the minimum value in the medical examination data of the medical examination item 2 is the medical examination data at 8:00, while 8:00 is neither the latest time 12:00 with corresponding medical examination data in the initial time period, nor the earliest time 3:00 with corresponding medical examination data in the initial time period, and then the time point of the minimum value is determined as the split time point.

c: partitioning by means of said partitioning module 305 the medical examination data in the initial time period at the split time point, so as to obtain the medical examination data in the target time period.

For example, the initial time period contains medical examination data at 3:00, medical examination data at 4:00, medical examination data at 7:00, medical examination data at 8:00, medical examination data at 9:00, and medical examination data at 12:00, and split time points are 4:00 and 8:00, then splitting yields target time periods of 3:00 to 4:00, 4:00 to 8:00, and 8:00 to 12:00.

d: splitting the medical examination data in the initial time period equally if the time point of the maximum value in the medical examination data corresponding to the medical examination item with the largest variance is the latest time point in the initial time period or the earliest time point in the initial time period, and the time point of the minimum value in the medical examination data corresponding to the medical examination item with the largest variance is the latest time point in the initial time period or the earliest time point in the initial time period, so as to obtain medical examination data in two target time periods.

For example, the medical examination item with the largest variance in the initial time period is medical examination item 2, the initial time period contains medical examination data at 3:00, medical examination data at 4:00, medical examination data at 7:00, medical examination data at 8:00, medical examination data at 9:00, and medical examination data at 12:00, the maximum value in the corresponding medical examination data of the medical examination item 2 is the medical examination data at 3:00, and the minimum value in the corresponding medical examination data of the medical examination item 2 is the medical examination data at 12:00, then the target time periods are 3:00 to 7:00 and 8:00 to 12:00.

S203: determining by means of said determination module 303 the medical examination data in the initial time period as the medical examination data in the target time period, if the variance of the medical examination data corresponding to all the medical examination items in the initial time period is less than or equal to the preset variance.

S107: inputting by means of said input module 304 the medical examination data in each target time period into a disease index determination model corresponding to the rare disease type, respectively, so as to obtain the disease index corresponding to each target time period, wherein the disease index determination model is trained with medical examination data samples and corresponding disease index labels.

In embodiments of the present invention, each rare disease type correspondingly has one disease index determination model. The disease index determination model is a deep learning model trained in advance with medical examination data samples of patients in a target time period corresponding to the rare diseased type and corresponding disease index labels, taking the medical examination data samples as input, and the disease index as output. A higher disease index indicates a more severe condition of the patient.

S108: generating by means of a generation module 306 a target trend polyline by taking the target time period as abscissa (horizontal axis), and corresponding disease index as ordinate (vertical axis).

S109: calculating by means of a calculation module 307 a similarity between the target trend polyline and each reference trend polyline, wherein the reference trend polyline is generated based on disease indexes for the patient with the rare disease type in multiple time periods.

In embodiments of the present invention, each reference trend polyline is corresponding to an actual progression trend. Progression trends include improving trends, deteriorating trends, and stable trends. The improving trends further include a slowly improving trend and a rapidly improving trend; the deteriorating trends further include a slowly deteriorating trend and a rapidly deteriorating trend. The reference trend polyline is generated based on disease index for the patient with the rare disease type corresponding to each historical time period; and the actual progression trend corresponding to the reference trend polyline is manually determined based on actual disease index development of the patient during a preset interval following each time period.

The similarity between the target trend polyline and each reference trend polyline is calculated through steps as follows.

Step 1: if the target trend polyline is longer than the reference trend polyline, trimming a leading section of the target trend polyline, so as to allow the remaining target trend polyline and the reference trend polyline to be of equal length.

Step 2: if the target trend polyline is shorter than the reference trend polyline, trimming a leading section of the reference trend polyline, so as to allow the remaining target trend polyline and the reference trend polyline to be of equal length.

Step 3: calculating by means of a calculation module 307 the similarity between the target trend polyline and each reference trend polyline based on the trimmed target trend polyline and reference trend polyline through a formula as follows:
$d = \frac{1}{\sqrt{{\sum }_{i = 1}^{n} {\left({h}_{i}-{g}_{i}\right)}^{2}}} ,$
wherein d is similarity between the target trend polyline and the reference trend polyline, n is length of the target trend polyline, hᵢ is disease index of a point with abscissa i in the trimmed target trend polyline, and gᵢ is disease index of a point with abscissa i in the reference trend polyline.

Step 4: calculating by means of a calculation module 307 the similarity between the target trend polyline and the reference trend polyline through a formula as follows if the target trend polyline and the reference trend polyline are of equal length:
$d = \frac{1}{\sqrt{{\sum }_{i = 1}^{n} {\left({z}_{i}-{g}_{i}\right)}^{2}}} ,$
wherein d is similarity between the target trend polyline and the reference trend polyline, n is length of the target trend polyline, hᵢ is disease index of a point with abscissa i in the target trend polyline, and gᵢ is disease index of a point with abscissa i in the reference trend polyline.

S110: determining by means of said determination module 303 the progression trend corresponding to the rare disease patient based on the similarity.

In embodiments of the present invention, the progression trend corresponding to the rare disease patient refers to a progression trend within a future preset time period.

Specifically, the progression trend corresponding to the rare disease patient is determined through steps as follows.

Step 1: determining by means of said determination module 303 an actual progression trend corresponding to a reference trend polyline with the maximum similarity, if a maximum value of the similarity is greater than or equal to a preset similarity, as the progression trend corresponding to the target rare disease patient.

Step 2: calculating, by means of a calculation module 307 if the maximum value of the similarity is less than the preset similarity and target points with a tangent slope of 0 exist in the target trend polyline, a difference between a disease index corresponding to a temporally latest target point and a disease index at a temporally latest point in the target trend polyline.

Herein, if the maximum value of the similarity is less than the preset similarity, the target trend polyline is determined as a reference trend polyline, which can increase the number of reference trend polylines, and improve a prediction accuracy rate.

Step 3: determining by means of said determination module 303 the progression trend corresponding to the rare disease patient as an improving trend if the difference is greater than 0.

In embodiments of the present invention, if the difference is greater than a first preset difference, the progression trend corresponding to the rare disease patient is a rapidly improving trend; and if the difference is greater than 0 and less than the first preset difference, the progression trend corresponding to the rare disease patient is a slowly improving trend.

Step 4: determining by means of said determination module 303 the progression trend corresponding to the rare disease patient as a deteriorating trend if the difference is less than 0.

In embodiments of the present invention, if the difference is less than a second preset difference, the progression trend corresponding to the rare disease patient is the rapidly deteriorating trend; and if the difference is less than 0 and greater than the second preset difference, the progression trend corresponding to the rare disease patient is the slowly deteriorating trend.

Step 5: determining by means of said determination module 303 the progression trend corresponding to the rare disease patient as a stable trend if the difference is equal to 0,.

Embodiments of the present invention provide a computer-implemented method for predicting the progression trend of the rare disease. The computer-implemented method includes: partitioning by means of said partitioning module 305 all medical examination data based on data characteristics of all the medical examination data in the current time period, so as to obtain medical examination data in multiple target time periods; inputting by means of said input module 304 the medical examination data in each target time period into the disease index determination model corresponding to the rare disease type, respectively, so as to obtain the disease index corresponding to each target time period; generating by means of a generation module 306 the target trend polyline by taking the target time period as abscissa, and corresponding disease index as ordinate; calculating by means of a calculation module 307 the similarity between the target trend polyline and each reference trend polyline; and determining by means of said determination module 303 the progression trend for the rare disease type in the rare disease patient based on the similarity. The present invention enables accurate prediction of the progression trend of the rare disease.

Based on the same inventive concept, embodiments of the present invention further provide an apparatus for predicting a progression trend of a rare disease corresponding to the computer-implemented method for predicting the progression trend of the rare disease. As the principle of the apparatus in the embodiments of the present invention for solving the problem is similar to that of the computer-implemented method for predicting the progression trend of the rare disease in the embodiments of the present invention, reference can be made to the implementation of the computer-implemented method for the implementation of the apparatus, and repetitive parts will not be elaborated.

With reference to FIG. 3, it shows a schematic diagram of the apparatus for predicting the progression trend of the rare disease provided in embodiments of the present invention. The apparatus for predicting the progression trend of the rare disease includes:
an acquisition module 301, configured to acquire a rare disease type of a target rare disease patient and medical examination data in a current time period;
a judgment module 302, configured to judge, for each time point in the current time period, whether there are any missing medical examination items correspondingly at the time point based on medical examination data at the time point and a set of medical examination items corresponding to the rare disease type, where the missing medical examination items refer to those without medical examination data;
a determination module 303, configured to determine an imputation weight corresponding to each missing medical examination item at the time point, if there are missing medical examination items correspondingly at the time point, based on a medical examination item association relationship corresponding to the rare disease type and the medical examination data at the time point, where the medical examination item association relationship includes other medical examination items associated with each medical examination item;
the determination module 303 being further configured to determine a missing medical examination item with a largest imputation weight as a target missing medical examination item at the time point;
an input module 304, configured to input, within the medical examination data at the time point, medical examination data corresponding to other medical examination items associated with the target missing medical examination item at the time point into an imputation model corresponding to the rare disease type and the target missing medical examination item, so as to obtain medical examination data for the target missing medical examination item at the time point; and return to judge, based on the medical examination data at the time point and the set of medical examination items corresponding to the rare disease type, whether there are any missing medical examination items correspondingly at the time point, so as to continue execution;
a partitioning module 305, configured to partition all medical examination data based on data characteristics of all the medical examination data in the current time period, so as to obtain medical examination data in multiple target time periods;
the input module 304 being further configured to input the medical examination data in each target time period into a disease index determination model corresponding to the rare disease type, respectively, so as to obtain the disease index corresponding to each target time period, wherein the disease index determination model is trained with medical examination data samples and corresponding disease index labels;
a generation module 306, configured to generate a target trend polyline by taking the target time period as abscissa, and corresponding disease index as ordinate;
a calculation module 307, configured to calculate a similarity between the target trend polyline and each reference trend polyline, wherein the reference trend polyline is generated based on disease indexes for the patient with the rare disease type in multiple time periods; and
the determination module 303 being further configured to determine the progression trend for the rare disease type in the rare disease patient based on the similarity.

In a possible embodiment, the determination module 303 is specifically configured to acquire historical medical examination data corresponding to each medical examination item in the set of medical examination items from a plurality of patients with the rare disease type at each historical time point; calculate an initial correlation coefficient between each two medical examination items of each patient based on all historical medical examination data corresponding to each patient; calculate, for each two medical examination items, a mean of initial correlation coefficients of all patients for the two medical examination items, so as to obtain a target correlation coefficient between the two medical examination items; and determine that an association relationship exists between the two medical examination items if the target correlation coefficient between the two medical examination items is greater than or equal to a preset correlation coefficient.

In a possible embodiment, the determination module 303 is specifically configured to substitute historical medical examination data of each two medical examination items of the patient into a following formula, so as to calculate the initial correlation coefficient between each two medical examination items of the patient:
$dCor \left(X, Y\right) = \frac{{dCor}^{2} \left(X, Y\right)}{\sqrt{dVar \left(X\right) ⋅ dVar \left(Y\right)}} ,$
wherein dCor(X,Y) is an initial correlation coefficient between historical medical examination data of a first medical examination item X in two medical examination items and historical medical examination data of a second medical examination item Y in the two medical examination items of the patient, dCor²(X,Y) is a distance covariance between the historical medical examination data of the first medical examination item X and the historical medical examination data of the second medical examination item Y of the patient, dVar(X) is a distance variance of the historical medical examination data of the first medical examination item X of the patient, and dVar(Y) is a distance variance of the historical medical examination data of the second medical examination item Y of the patient.

In a possible embodiment, the partitioning module 305 is specifically configured to partition all the medical examination data according to a preset partitioning interval, so as to obtain medical examination data in multiple initial time periods; partition the medical examination data within each initial time period based on medical examination data of a medical examination item with the largest variance within that initial time period, for each initial time period, if a variance of medical examination data corresponding to any medical examination item within that initial time period is greater than a preset variance, so as to obtain medical examination data within the target time period; and determine the medical examination data in the initial time period as the medical examination data in the target time period, if the variance of the medical examination data corresponding to all medical examination items in the initial time period is less than or equal to a preset variance.

In a possible embodiment, the partitioning module 305 is specifically configured to determine a time point of a maximum value as a split time point if the time point of the maximum value in the corresponding medical examination data of the medical examination item with the largest variance is neither a latest time point in the initial time period nor an earliest time point in the initial time period; determine a time point of a minimum value as a split time point if the time point of the minimum value in the corresponding medical examination data of the medical examination item with the largest variance is neither a latest time point in the initial time period nor an earliest time point in the initial time period; partition the medical examination data in the initial time period at the split time point, so as to obtain the medical examination data in the target time period; and split the medical examination data in the initial time period equally if the time point of the maximum value in the medical examination data corresponding to the medical examination item with the largest variance is the latest time point in the initial time period or the earliest time point in the initial time period, and the time point of the minimum value in the medical examination data corresponding to the medical examination item with the largest variance is the latest time point in the initial time period or the earliest time point in the initial time period, so as to obtain medical examination data in two target time periods.

In a possible embodiment, the determination module 303 is specifically configured to determine an actual progression trend corresponding to a reference trend polyline with a maximum similarity, if a maximum value of the similarity is greater than or equal to a preset similarity, as the progression trend corresponding to the target rare disease patient; calculate, if the maximum value of the similarity is less than the preset similarity and target points with a tangent slope of 0 exist in the target trend polyline, a difference between a disease index corresponding to a temporally latest target point and a disease index at a temporally latest point in the target trend polyline; determine the progression trend corresponding to the rare disease patient as an improving trend if the difference is greater than 0; and determine the progression trend corresponding to the rare disease patient as a deteriorating trend if the difference is less than 0.

In a possible embodiment, if the maximum value of similarity is less than the preset similarity, the determination module 303 is further configured to determine the target trend polyline as a reference trend polyline.

Embodiments of the present invention provide the apparatus for predicting the progression trend of the rare disease. The apparatus of the present invention enables accurate prediction of the progression trend of the rare disease.

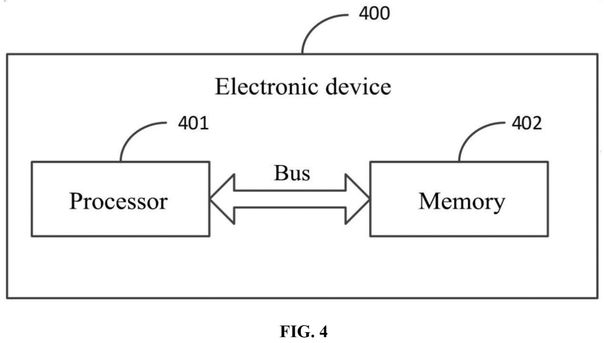

With reference to FIG. 4, embodiments of the present invention provide an electronic device 400, including: a processor 401, a memory 402, and a bus, wherein the memory 402 stores machine-readable instructions executable by the processor 401. When the electronic device is running, the processor 401 and the memory 402 communicate via the bus, and the processor 401 executes the machine-readable instructions, so as to execute the steps of the above computer-implemented method for predicting the progression trend of the rare disease.

Specifically, the memory 402 and processor 401 in the above can be general-purpose memory and processor, without specific limitation herein. The processor 401, when running a computer program stored in the memory 402, is capable of executing the above computer-implemented method for predicting the progression trend of the rare disease.

Corresponding to the above computer-implemented method for predicting the progression trend of the rare disease, embodiments of the present invention further provide a computer-readable storage medium, on which a computer program is stored, and the computer program, when run by a processor, executes the steps of the above computer-implemented method for predicting the progression trend of the rare disease.

It would be clear to those skilled in the art that, for the convenience and conciseness of the description, reference can be made to corresponding processes in the computer-implemented method embodiments for specific operation processes of the system and apparatus described in the above, which will not be elaborated herein. In the several embodiments provided in the present invention, it should be understood that the system, apparatus, and computer-implemented method disclosed can be implemented in other ways. The apparatus embodiments described in the above are merely exemplary, for example, the modules are merely divided according to logical functions, but they may be divided in other ways in practical implementation. For another example, a plurality of modules or components can be combined or integrated into another system, or some features can be omitted, or not executed. In addition, a mutual coupling, direct coupling or communication connection shown or discussed can be an indirect coupling or communication connection through some communication interfaces, means or modules, and can be in an electrical form, a mechanical form or other forms.

The modules described as separate components may or may not be physically separated, and the components displayed as modules may or may not be physical units, meaning that they can be located in one place or distributed across multiple network units. The objective of the solutions of the embodiments can be realized by selecting some or all of the units thereof as actually required.

Besides, various functional units in various embodiments of the present invention can be integrated into one processing unit, various units can exist physically independently, or two or more units can also be integrated into one unit.

The functionality, if implemented in the form of software functional unit and sold or used as standalone product, can be stored in a non-volatile, computer-readable storage medium executable by a processor. Based on such understanding, the technical solutions of the present invention in essence or parts making contribution to the prior art or parts of the technical solutions can be embodied in form of a software product. This computer software product is stored in a storage medium, and includes several instructions for causing a computer device (which may be a personal computer, a server or a network device, etc.) to execute all or some of the steps of the information processing computer-implemented method of various embodiments of the present invention. The aforementioned storage medium includes various media capable of storing program code, such as USB flash drives, portable hard drives, ROM, RAM, magnetic disks or optical disks.

The above-mentioned are merely for specific embodiments of the present invention, but the scope of protection of the present invention is not limited thereto. Modifications or substitutions readily conceivable to those skilled in the art within the technical scope disclosed in the present invention should be covered within the scope of protection of the present invention Therefore, the scope of protection of the present invention should be determined by the scope of protection of the claims.

## Claims

1. A computer-implemented method for predicting a progression trend of a rare disease, **characterized by** comprising the steps of:
acquiring by means of an acquisition module (301) a rare disease type of a target rare disease patient and medical examination data in a current time period;
judging by means of a judgment module (302), for each time point in the current time period, whether there are any missing medical examination items correspondingly at the time point based on medical examination data at the time point and a set of medical examination items corresponding to the rare disease type, wherein the missing medical examination items are medical examination items without medical examination data;
determining by means of a determination module (303) an imputation weight corresponding to each missing medical examination item at the time point, if there are missing medical examination items correspondingly at the time point, based on a medical examination item association relationship corresponding to the rare disease type and the medical examination data at the time point, wherein the medical examination item association relationship comprises other medical examination items associated with each medical examination item;
determining by means of said determination module (303) a missing medical examination item with a largest imputation weight as a target missing medical examination item at the time point;
inputting by means of an input module (304), within the medical examination data at the time point, medical examination data corresponding to the other medical examination items associated with the target missing medical examination item at the time point into an imputation model corresponding to the rare disease type and the target missing medical examination item, so as to obtain medical examination data for the target missing medical examination item at the time point; and returning to the step of judging by means of said judgment module (302) whether there are any missing medical examination items correspondingly at the time point based on the medical examination data at the time point and the set of medical examination items corresponding to the rare disease type, so as to continue execution;
partitioning by means of a partitioning module (305) all the medical examination data based on data characteristics of all the medical examination data in the current time period, so as to obtain medical examination data in multiple target time periods;
inputting by means of said input module (304) the medical examination data in each target time period into a disease index determination model corresponding to the rare disease type, respectively, so as to obtain a disease index corresponding to each target time period, wherein the disease index determination model is trained with medical examination data samples and corresponding disease index labels;
generating by means of a generation module (306) a target trend polyline by taking the target time period as abscissa, and corresponding disease index as ordinate;
calculating by means of a calculation module (307) a similarity between the target trend polyline and each reference trend polyline, wherein the reference trend polyline is generated based on disease indexes for a patient with the rare disease type in multiple time periods; and
determining by means of said determination module (303) the progression trend for the rare disease type in the rare disease patient based on the similarity.

2. The computer-implemented method for predicting the progression trend of the rare disease according to claim 1, wherein the medical examination item association relationship corresponding to the rare disease type is determined through steps of:
acquiring by means of said acquisition module (301) historical medical examination data corresponding to each medical examination item in the set of medical examination items from a plurality of patients with the rare disease type at each historical time point;
calculating by means of said calculation module (307) an initial correlation coefficient between each two medical examination items of each patient based on all the historical medical examination data corresponding to each patient;
for each two medical examination items, calculating by means of said calculation module (307) a mean of initial correlation coefficients of all the patients for the two medical examination items, so as to obtain a target correlation coefficient between the two medical examination items; and
determining, by means of said determination module (303) if the target correlation coefficient between the two medical examination items is greater than or equal to a preset correlation coefficient, that an association relationship exists between the two medical examination items.

3. The computer-implemented method for predicting the progression trend of the rare disease according to claim 2, wherein the step of calculating by means of said calculation module (307) the initial correlation coefficient between each two medical examination items of each patient based on all the historical medical examination data corresponding to each patient comprises:
substituting historical medical examination data of each two medical examination items of the patient into a following formula, to calculate the initial correlation coefficient between each two medical examination items of the patient: $dCor \left(X, Y\right) = \frac{{dCor}^{2} \left(X, Y\right)}{\sqrt{dVar \left(X\right) ⋅ dVar \left(Y\right)}} ,$
wherein dCor(X,Y) is an initial correlation coefficient between historical medical examination data of a first medical examination item X in the two medical examination items and historical medical examination data of a second medical examination item Y in the two medical examination items of the patient, dCor²(X,Y) is a distance covariance between the historical medical examination data of the first medical examination item X and the historical medical examination data of the second medical examination item Y of the patient, dVar(X) is a distance variance of the historical medical examination data of the first medical examination item X of the patient, and dVar(Y) is a distance variance of the historical medical examination data of the second medical examination item Y of the patient.

4. The computer-implemented method for predicting the progression trend of the rare disease according to claim 1, wherein the step of partitioning by means of said partitioning module (305) all the medical examination data based on the data characteristics of all the medical examination data in the current time period, so as to obtain the medical examination data in the multiple target time periods comprises:
partitioning by means of said partitioning module (305) all the medical examination data according to a preset partitioning interval, so as to obtain medical examination data in multiple initial time periods;
partitioning, by means of said partitioning module (305) for each initial time period, the medical examination data within the initial time period based on the medical examination data of a medical examination item with a largest variance within the initial time period, if a variance of the medical examination data corresponding to any medical examination item within the initial time period is greater than a preset variance, so as to obtain the medical examination data within the target time period; and
determining by means of said determination module (303) the medical examination data in the initial time period as the medical examination data in the target time period, if the variance of the medical examination data corresponding to all the medical examination items in the initial time period is less than or equal to the preset variance.

5. The computer-implemented method for predicting the progression trend of the rare disease according to claim 4, wherein the step of partitioning by means of said partitioning module (305) the medical examination data within the initial time period based on the medical examination data of the medical examination item with the largest variance within the initial time period, so as to obtain the medical examination data within the target time period comprises:
determining by means of said determination module (303) a time point of a maximum value as a split time point if the time point of the maximum value in the medical examination data of the medical examination item with the largest variance is neither a latest time point in the initial time period nor an earliest time point in the initial time period;
determining by means of said determination module (303) a time point of a minimum value as a split time point if the time point of the minimum value in the medical examination data of the medical examination item with the largest variance is neither a latest time point in the initial time period nor an earliest time point in the initial time period;
partitioning by means of said partitioning module (305) the medical examination data in the initial time period at the split time point, so as to obtain the medical examination data in the target time period; and
splitting the medical examination data in the initial time period equally if the time point of the maximum value in the medical examination data corresponding to the medical examination item with the largest variance is the latest time point in the initial time period or the earliest time point in the initial time period, and the time point of the minimum value in the medical examination data corresponding to the medical examination item with the largest variance is the latest time point in the initial time period or the earliest time point in the initial time period, so as to obtain medical examination data in two target time periods.

6. The computer-implemented method for predicting the progression trend of the rare disease according to claim 5, wherein the step of determining by means of said determination module (303) the progression trend for the rare disease type in the rare disease patient based on the similarity comprises steps of:
determining by means of said determination module (303) an actual progression trend corresponding to a reference trend polyline with a maximum similarity, if a maximum value of the similarity is greater than or equal to a preset similarity, as the progression trend corresponding to the target rare disease patient;
calculating, by means of a calculation module (307) if the maximum value of the similarity is less than the preset similarity and target points with a tangent slope of 0 exist in the target trend polyline, a difference between a disease index corresponding to a temporally latest target point and a disease index at a temporally latest point in the target trend polyline;
determining by means of said determination module (303) the progression trend corresponding to the rare disease patient as an improving trend if the difference is greater than 0; and
determining by means of said determination module (303) the progression trend corresponding to the rare disease patient as a deteriorating trend if the difference is less than 0.

7. The computer-implemented method for predicting the progression trend of the rare disease according to claim 6, wherein if the maximum value of the similarity is less than the preset similarity, the computer-implemented method further comprises:
determining by means of said determination module (303) the target trend polyline as a reference trend polyline.

8. The computer-implemented method for predicting the progression trend of the rare disease according to claim 1, wherein the step of determining by means of said determination module (303) an imputation weight corresponding to each missing medical examination item at the time point based on a medical examination item association relationship corresponding to the rare disease type and the medical examination data at the time point comprises:
for each missing medical examination item, the number of other medical examination items associated with the missing medical examination item and having corresponding medical examination data is counted based on the medical examination data at the time point, to obtain the imputation weight corresponding to the missing medical examination item.

9. The computer-implemented method for predicting the progression trend of the rare disease according to claim 1, wherein the imputation model is a deep learning model trained in advance with medical examination data samples of other medical examination items associated with the missing medical examination item and corresponding medical examination data labels from the same patient at the same time point.

10. The computer-implemented method for predicting the progression trend of the rare disease according to claim 1, wherein the disease index determination model is a deep learning model trained in advance with medical examination data samples of patients in a target time period corresponding to the rare diseased type and corresponding disease index labels.

11. The computer-implemented method for predicting the progression trend of the rare disease according to claim 1, wherein the step of calculating by means of said calculation module (307) a similarity between the target trend polyline and each reference trend polyline comprises:
if the target trend polyline is longer than the reference trend polyline, trimming a leading section of the target trend polyline, so as to allow the remaining target trend polyline and the reference trend polyline to be of equal length;
if the target trend polyline is shorter than the reference trend polyline, trimming a leading section of the reference trend polyline, so as to allow the remaining target trend polyline and the reference trend polyline to be of equal length; and
calculating by means of said calculation module (307) the similarity between the target trend polyline and each reference trend polyline based on the trimmed target trend polyline and reference trend polyline through a formula as follows: $d = \frac{1}{\sqrt{{\sum }_{i = 1}^{n} {\left({h}_{i}-{g}_{i}\right)}^{2}}} ,$
wherein d is similarity between the target trend polyline and the reference trend polyline, n is length of the target trend polyline, hᵢ is disease index of a point with abscissa i in the trimmed target trend polyline, and gᵢ is disease index of a point with abscissa i in the reference trend polyline.

12. The computer-implemented method for predicting the progression trend of the rare disease according to claim 6, wherein
if the difference is greater than a first preset difference, the progression trend corresponding to the rare disease patient is a rapidly improving trend; and
if the difference is greater than 0 and less than the first preset difference, the progression trend corresponding to the rare disease patient is a slowly improving trend; and/or
if the difference is less than a second preset difference, the progression trend corresponding to the rare disease patient is the rapidly deteriorating trend; and
if the difference is less than 0 and greater than the second preset difference, the progression trend corresponding to the rare disease patient is the slowly deteriorating trend.

13. An apparatus for predicting a progression trend of a rare disease, **characterized by** comprising:
an acquisition module (301), configured to acquire a rare disease type of a target rare disease patient and medical examination data in a current time period;
a judgment module (302), configured to judge, for each time point in the current time period, whether there are any missing medical examination items correspondingly at the time point based on medical examination data at the time point and a set of medical examination items corresponding to the rare disease type, wherein the missing medical examination items are medical examination items without medical examination data;
a determination module (303), configured to determine an imputation weight corresponding to each missing medical examination item at the time point, if there are missing medical examination items correspondingly at the time point, based on a medical examination item association relationship corresponding to the rare disease type and the medical examination data at the time point, wherein the medical examination item association relationship comprises other medical examination items associated with each medical examination item;
the determination module (303) being further configured to determine a missing medical examination item with a largest imputation weight as a target missing medical examination item at the time point;
an input module (304), configured to input, within the medical examination data at the time point, medical examination data corresponding to other medical examination items associated with the target missing medical examination item at the time point into an imputation model corresponding to the rare disease type and the target missing medical examination item, so as to obtain medical examination data for the target missing medical examination item at the time point; and return to judge whether there are any missing medical examination items correspondingly at the time point based on the medical examination data at the time point and the set of medical examination items corresponding to the rare disease type, so as to continue execution;
a partitioning module (305), configured to partition all the medical examination data based on data characteristics of all the medical examination data in the current time period, so as to obtain medical examination data in multiple target time periods;
the input module (304) being further configured to input the medical examination data in each target time period into a disease index determination model corresponding to the rare disease type, respectively, so as to obtain the disease index corresponding to each target time period, wherein the disease index determination model is trained with medical examination data samples and corresponding disease index labels;
a generation module (306), configured to generate a target trend polyline by taking the target time period as abscissa, and corresponding disease index as ordinate;
a calculation module (307), configured to calculate a similarity between the target trend polyline and each reference trend polyline, wherein the reference trend polyline is generated based on disease indexes for a patient with the rare disease type in multiple time periods; and
the determination module (303) being further configured to determine the progression trend for the rare disease type in the rare disease patient based on the similarity,

14. An electronic device, **characterized by** comprising: a processor, a storage medium, and a bus, wherein the storage medium stores machine-readable instructions executable by the processor, when the electronic device is running, the processor and the storage medium communicate via the bus, and the processor executes the machine-readable instructions, so as to execute the steps of the computer-implemented method for predicting the progression trend of the rare disease according to any one of claims 1 to 7.

15. A computer-readable storage medium, wherein a computer program is stored on the computer-readable storage medium, and the computer program, when run by a processor, executes the steps of the computer-implemented method for predicting the progression trend of the rare disease according to any one of claims 1 to 7.
